# FASCICULE DE BREVET EUROPEEN

(11) **EP 2 850 213 B1**
(45) Date de publication et mention de la délivrance du brevet: **07.06.2017**
(21) Numéro de dépôt: 13727290.2
(22) Date de dépôt: 15.05.2013
(51) Int. Cl.: A23L 29/30, C12R 1/425

(54) **SOUCHE PRODUCTRICE DE TURANOSE ET UTILISATIONS**
TURANOSE PRODUZIERENDER STAMM UND VERWENDUNGEN DAVON
STRAIN PRODUCING TURANOSE AND USES THEREOF

(30) Priorité: 16.05.2012 FR 1254484
(43) Date de publication de la demande: 25.03.2015
(73) Titulaire: Roquette Frères, 62136 Lestrem (FR)
(72) Inventeur: DEFRETIN, Sophie, F-62400 Bethune (FR); RAECKELBOOM, Damien, F-62136 Lestrem (FR)
(74) Mandataire: Gallois, Valérie
(86) Numéro de dépôt international: PCT/FR2013/051054
(87) Numéro de publication internationale: WO 2013/171424

(56) Documents cités:
- WO-A1-2007/107295
- REN WANG ET AL: "Development of an efficient bioprocess for turanose production by sucrose isomerisation reaction of amylosucrase", FOOD CHEMISTRY, ELSEVIER LTD, NL, vol. 132, no. 2, 17 novembre 2011 (2011-11-17), pages 773-779, XP028350500, ISSN: 0308-8146, DOI: 10.1016/J.FOODCHEM.2011.11.035 [extrait le 2011-11-17]
- DATABASE WPI Week 201048 Thomson Scientific, London, GB; AN 2010-C73136 XP002691248, & KR 2010 0022260 A (LG HOUSEHOLD & HEALTHCARE LTD) 2 mars 2010 (2010-03-02)

## Description

### Domaine de l'invention

La présente invention est relative à une souche productrice de turanose et ses utilisations.

### Arrière-plan technologique de l'invention

Le turanose est un isomère du saccharose comprenant une unité glucose et une unité fructose lié par une liaison α(1→3). C'est un sucre typique du miel, dans lequel il est présent en faible quantité généralement comprise entre 0 et 3 %.

Il peut par exemple être produit par hydrolyse partielle du mélézitose, produisant ainsi un mélange équimolaire de glucose et de turanose (CS240545). En outre, il peut également être produit par l'action d'une cyclomaltodextrine glucanotransférase de *Bacillus stearothermophilus* sur un mélange d'amidon et de fructose (JP5252974/93 ; Shibuya et al, 2004, J. Appl. Glycosci., 51, 223-227). Le rendement n'est que de 45 % et la méthode comprend deux étapes enzymatiques.

Enfin, il a été proposé d'utiliser une amylosucrase recombinante issue de *Neisseria polysaccharea* (NpAS) pour convertir du saccharose en turanose (Wang et al, 2012, Food Chemistry, 132, 773-779). Le rendement est de 56 % et la méthode nécessite une enzyme recombinante.

Il est connu de l'homme du métier que le turanose :
- présente un pouvoir sucrant relativement faible de 0,5 (la valeur 1 étant attribuée au saccharose), et une faible cariogénicité,
- peut être facilement cristallisé,
- est fortement soluble.

En outre, le turanose est un inhibiteur de l'α-glucosidase, utile dans le diagnostic de la maladie de Pompe.

Il présente également un intérêt dans les domaines alimentaires, cosmétiques, pharmaceutiques et diagnostiques.

Le turanose est également une molécule message. Avec d'autres oses, le turanose a ainsi été utilisé pour mimer les différentes voies de signalisation du saccharose dans la photosynthèse.

Le turanose peut-être également impliqué dans les mécanismes de défense des plantes avec production de substances défensives par activation des MAPKs (Mitogen-Activated Protein Kinases).

Ainsi, des méthodes alternatives de production de turanose sont utiles et recherchées, en particulier des méthodes compétitives au niveau industriel.

### Résumé de l'invention

Soucieuse de mettre au point un procédé de production bien plus efficace et bien moins couteux que ceux décrits dans l'état de la technique, la société Demanderesse a, au cours de ses recherches, identifié une nouvelle souche présentant la capacité de produire du turanose.

**La présente invention est donc relative à une souche bactérienne de *Serratia plymuthica* capable de produire du turanose. Cette souche a été déposée le 7 mars 2012 auprès de la CNCM sous le numéro I-4604.**

Elle est également relative à une souche de *Serratia plymuthica* caractérisée en ce qu'elle est capable de produire du turanose, et qu'elle est obtenue à partir de la souche I-4604 par culture, mutagenèse ou modification génétique de celle-ci.

De préférence, la souche de *Serratia plymuthica* selon la présente invention est capable de produire du turanose avec un rendement massique turanose/saccharose d'au moins 20%, 30%, 40% ou 50 %.

Elle concerne une méthode de production de turanose comprenant une culture de la souche selon la présente invention et la récupération du turanose, et facultativement la purification du turanose.

De préférence, la souche est cultivée à pH compris entre 5,5 et 7, une aération de 0,5 à 1,5 vvm, dans des conditions d'agitation comprise entre 250 et 700 rpm, et à une température de 25 à 38 °C. De manière en plus préférée, la souche est cultivée à un pH maintenu à 6, une température de 27 à 30°C, des conditions d'agitation comprises entre 250 et 350 rpm, et une aération de 1 vvm.

De préférence, la concentration de saccharose de départ dans le milieu de production est comprise entre 100 et 300 g/L, de préférence de 200 g/L.

Dans un mode de réalisation particulier, le milieu de production comprend 2 à 6 g/L d'extrait de levure ou 10 à 30 g/L de liqueur de maïs macéré (tel que le SOLULYS^{®} 048E commercialisé par la société Demanderesse), de préférence environ 4 g/L d'extrait de levure. De préférence, la purification du turanose comprend une étape de centrifugation du mout de fermentation, des étapes de traitement au noir en poudre et de filtration du surnageant, une étape de déminéralisation, une étape d'ultrafiltration avec un seuil de coupure à 1 kD, puis une étape de cristallisation du filtrat.

La présente demande décrit une composition riche en turanose susceptible d'être obtenue ou obtenue par le procédé selon la présente invention. De préférence, elle comprend au moins 80 % de turanose en poids total de DP2, 0,01 à 10 % de tréhalulose en poids total de DP2 et 0,01 à 5 % d'isomaltulose en poids total de DP2.

La présente demande décrit une méthode de préparation d'une composition alimentaire comprenant la fourniture de turanose obtenu par la méthode selon la présente invention et l'incorporation du turanose obtenu dans la composition alimentaire. Elle est également relative à une méthode de préparation d'une composition alimentaire comprenant la fourniture d'une composition riche en turanose obtenue par la méthode selon la présente invention et l'incorporation de la composition obtenue dans la composition alimentaire

Enfin, la présente demande décrit une méthode de préparation d'une composition riche en turanose susceptible d'être obtenue ou obtenue par le procédé selon la présente invention destinée aux domaines alimentaires, cosmétiques, pharmaceutiques, diagnostiques et phytosanitaires.

### Description détaillée de l'invention

De manière tout à fait surprenant, le Demandeur a réussi à identifier et isoler une souche capable de produire du turanose de façon efficace parmi un très grand nombre de microorganismes issus d'une campagne de prélèvement de terre.

La souche identifiée est une bactérie *Serratia plymuthica* et elle est appelée ici I-4604. Elle a été déposée le 7 mars 2012 auprès de la CNCM sous le numéro I-4604. Cette souche pourra être désignée « I-4604 » ultérieurement dans la présente demande.

Cette souche présente la propriété intéressante de produire du turanose en grande quantité. En effet, elle permet l'obtention du turanose avec un rendement massique turanose/saccharose de plus de 35 %. Plus précisément, un rendement moyen turanose/saccharose d'environ 38 % a été observé lorsqu'une base mélasse a été utilisée. Ce rendement augmente à plus de 50 % lorsqu'une base saccharose est utilisée.

Cette souche est tout à fait originale quant à sa capacité de produire du turanose. En effet, les inventeurs ont également testé trois souches du même genre, à savoir *Serratia plymuthica* ATCC 15928, *Serratia ficaria* GRIMONT 4024, et *Serratia ficaria* DSM 4569. Aucune des souches testées à titre de comparaison n'a montré la moindre capacité à produire du turanose.

Ainsi, la présente invention est relative à la souche I-4604 et à une bactérie dérivée de cette souche, par exemple par culture, génie génétique ou mutagenèse de celle-ci, qui conserve la propriété de produire du turanose. La mutagenèse peut être dirigée et/ou aléatoire. En particulier, les souches *Serratia plymuthica* selon la présente invention présentent la capacité de produire du turanose avec un rendement massique turanose/saccharose d'au moins 20 %, 30 %, 40 % ou 50 %. De préférence, le rendement est d'au moins 30 %.

La présente invention concerne une composition comprenant une souche de *Serratia plymuthica* selon la présente invention, en particulier la souche I-4604, et facultativement un milieu de culture. De préférence, le milieu de culture est approprié à la production de turanose par la souche de *Serratia plymuthica* selon la présente invention, en particulier la souche I-4604. Notamment, ce milieu comprend du saccharose. Idéalement, il comprend environ 100 à 300 g/L, environ 100 à 200 g/L ou environ 200 g/L de saccharose. Plus précisément, le milieu de culture peut comprendre du saccharose et ainsi que de l'extrait de levure et/ou de liqueur de maïs macéré (tel que le SOLULYS^{®} 048E commercialisé par la société Demanderesse).

Est entendu dans le présent document que le terme « environ » signifie plus ou moins 10 %, de préférence plus ou moins 5 %. Par exemple, pour une valeur de 100, « environ 100 » signifie entre 90 et 110, de préférence entre 95 et 105.

La présente invention concerne l'utilisation d'une souche de *Serratia plymuthica* selon la présente invention, en particulier la souche I-4604, dans une réaction de fermentation. En particulier, la présente invention concerne l'utilisation d'une souche de *Serratia plymuthica* selon la présente invention, en particulier la souche I-4604, pour la production de turanose.

Elle concerne en outre une méthode de production de turanose, comprenant une culture de la souche selon la présente invention et la récupération du turanose, et facultativement la purification du turanose. En particulier, la culture de la souche est faite en présence de saccharose dans des conditions de fermentation adaptées à la production de turanose.

De préférence, la souche a été soumise, avant l'étape de production ou fermentation, à une étape de préculture et de subculture. Les milieux de préculture et de subculture comprennent du saccharose, par exemple d'environ 50 à 150 g/L de saccharose, de préférence environ 100 g/L. Ces milieux comprennent en outre des nutriments. Notamment, ils peuvent comprendre des extraits de levure et/ou des liqueurs de maïs macéré (tel que le SOLULYS^{®} 048E commercialisé par la société Demanderesse).

Par exemple, ils peuvent comprendre 5-15 g/L d'extrait de levure et/ou 5 à 30 g/L de SOLULYS^{®} 048E. Notamment, ils peuvent comprendre soit environ 10-15 g/L d'extrait de levure, soit environ 10-15 g/L d'extrait de levure et environ 5 g/L de SOLULYS^{®} 048E, soit environ 30 g/L de SOLULYS^{®} 048E. Dans un mode de réalisation préféré, ils comprennent environ 10-15 g/L d'extrait de levure. Les étapes de préculture et de subculture sont réalisées à une température de 25 à 38 °C, de préférence de 27 à 30°C, et en particulier à environ 30°C. L'étape de préculture peut durer de 10 à 30 h, de préférence environ 15 à 25 h. L'étape de subculture peut durer de 5 à 25 h, de préférence environ 10 à 20 h. Le pH de départ peut être compris entre environ 5,5 et environ 7. Dans un mode de réalisation préféré, le pH de départ est d'environ 7. L'agitation peut être comprise entre environ 100 et 200 rpm (tour par minute). De préférence, elle est d'environ 150 et 170 rpm.

De préférence, le saccharose est présent dans le milieu de production à une concentration initiale comprise entre environ 100 et environ 300 g/L. De préférence, il est présent à une concentration initiale d'environ 100 à 200 g/L. Dans un mode de réalisation préféré, il est présent à une concentration initiale d'environ 200 g/L. Le saccharose peut être ajouté sous forme purifié ou sous forme de mélasse. Il peut être ajouté au milieu de culture en début de production ou de fermentation, plusieurs fois au cours de la production ou de fermentation ou en continue lors de production ou fermentation. Dans un mode de réalisation préféré, il est ajouté au milieu de culture en début de production ou de fermentation.

Concernant les conditions de pH, il a été déterminé, lors de l'optimisation de la production de turanose, que le pH pouvait être compris entre environ 5,5 et 7, de préférence entre environ 5,5 et environ 6,5, de manière encore plus préférée à environ 6. De préférence, le pH est maintenu pendant l'étape de production. Dans un mode de réalisation, le pH est maintenu à environ pH 6 pendant l'étape de production.

Concernant les conditions d'agitation, la culture est faite sous agitation, en particulier sous agitation entre environ 250 et environ 700 rpm, de préférence entre 300 et 500 rpm. L'agitation peut être faite à l'aide de barreau magnétique ou tout autre moyen connu de l'homme du métier. Dans un mode de réalisation, la culture est faite sous agitation à environ 300 rpm.

Concernant les conditions d'aération, la culture est faite avec une aération de 0,5 à 1,5 vvm (volume d'air par minute et par volume). Dans un mode de réalisation, la culture est faite avec une aération d'environ 1 vvm.

Concernant les conditions de température, la culture est faite à une température comprise entre environ 25 à environ 38 °C. Dans un mode de réalisation, la culture est faite à une température comprise entre 27 à 30°C.

Dans un mode de réalisation particulier, le milieu de production comprend 2 à 6 g/L d'extrait de levure ou 10 à 30 g/L de SOLULYS^{®} 048E, de préférence environ 4 g/L d'extrait de levure.

La durée de culture peut être déterminée par une durée maximale conditionnée par la consommation totale du saccharose. De préférence, la culture de production dure au moins 20 h, typiquement plus de 30 h. Dans un mode de réalisation préféré, la culture de production dure entre 30 et 50 h, de préférence environ 40 h.

De préférence, l'étape de production est réalisée en respectant une ou plusieurs conditions de fermentation telles que détaillées ci-dessus. De préférence, toutes les conditions sont respectées.

Après l'étape de fermentation, la biomasse peut être récupérée du milieu de fermentation par toute méthode connue en soi de l'homme du métier, par exemple la biomasse peut être extraite du fermenteur et simplement concentrée par microfiltration ou centrifugation, ou lavée par succession de concentrations-dilutions avec une solution aqueuse.

Après l'étape de récolte du turanose, la méthode peut comprendre une étape de purification du turanose. Cette étape de purification peut comprendre une étape de centrifugation du mout de fermentation, le surnageant étant récupéré. Ce surnageant peut faire l'objet d'étapes supplémentaires de purification. Ces étapes de purification peuvent être choisies parmi des étapes de traitement au noir de charbon, des étapes de filtration, des étapes d'ultrafiltration, des étapes de déminéralisation, des étapes de cristallisation et des combinaisons de celles-ci. Dans un mode de réalisation particulier, la purification comprend une étape de traitement au noir de charbon, une étape de filtration, une étape de déminéralisation, une étape d'ultrafiltration avec un seuil de coupure de 1 kD et une étape de cristallisation. Dans un mode de réalisation préféré, elle comprend ces étapes dans l'ordre d'apparition de la liste.

La présente demande décrit un turanose d'origine fermentaire susceptible d'être obtenu ou obtenu par fermentation de la souche de *Serratia plymuthica* selon la présente invention, en particulier la souche I-4604. Ainsi elle est relative au turanose susceptible d'être obtenu ou obtenu par la méthode de production selon la présente invention.

La présente demande décrit une composition riche en turanose susceptible d'être obtenu ou obtenu par fermentation de la souche de *Serratia plymuthica* selon la présente invention, en particulier la souche I-4604, ou autrement dit par la méthode de production selon la présente invention. Cette composition comprend au moins 80 % de turanose en poids total de DP2, de préférence au moins 85 %, 90 %, ou 95 %. Par DP2 est entendu « disaccharides ». La composition peut comprendre en outre du tréhalulose et/ou de l'isomaltulose. Le tréhalulose est de préférence présent dans la composition dans des proportions comprises entre 0,01 à 10 % en poids total de DP2. L'isomaltulose est de préférence présent dans la composition dans des proportions comprises entre 0,01 à 5 % en poids total de DP2. De préférence, la composition comprend du tréhalulose et de l'isomaltulose. D'autres impuretés peuvent également être présentes comme l'acide glycérique glucosylé, le fructose, l'acide 2-cétoglutarique, l'acide citrique, l'acide succinique, et/ou l'acide lactique glucosylé.

Le turanose est particulièrement utile dans l'industrie alimentaire. Notamment, il peut être incorporé dans des boissons, des confiseries, des barres de céréales, des produits chocolatés, etc... Ainsi, la demande décrit une composition alimentaire comprenant une composition riche en turanose susceptible d'être obtenu ou obtenu par fermentation de la souche de *Serratia plymuthica* selon la présente invention, en particulier la souche I-4604.

Enfin, la présente demande décrit une méthode de préparation d'une composition alimentaire comprenant la fourniture de turanose obtenu par la méthode selon la présente invention et l'incorporation du turanose obtenu dans la composition alimentaire. De préférence, la composition alimentaire est une boisson, une confiserie, une barre de céréales ou un produit chocolaté.

Cette composition peut également être mise à profit dans les domaines alimentaires, cosmétiques, pharmaceutiques, diagnostiques et phytosanitaires.

L'invention sera mieux comprise à l'aide des exemples qui suivent.

### Exemples

### Exemple 1 - Production de turanose à partir de saccharose par la souche I-4604 et comparaison avec des souches de même genre et espèce

La fermentation de turanose à partir de saccharose a été testée avec la souche identifiée dans la présente invention I-4604 et avec d'autres souches de même genre et espèce, notamment les souches suivantes :
*Serratia plymuthica* ATCC 15928
*Serratia ficaria* GRIMONT 4024
*Serratia ficaria* DSM 4569

### 1- Résultats

### 1.1- Fermentations réalisées avec la souche I-4604

| | **[Saccharose]_{c} *g.L⁻¹*** | **[Turanose]ₚ *g.L⁻¹*** | **[Isomaltulose]ₚ *g.L⁻¹*** | **Y_{Tur/Sac} %** | **Productivité *g.L⁻¹.h⁻¹*** |
|---|---|---|---|---|---|
| Base mélasse | 120,2 | 53,1 | ND | 37,69 | 1,36 |
| | 123 | 54,9 | ND | 38,19 | 1,41 |
| Base saccharose | **187,4** | **103,4** | **ND** | **46,37** | **2,66** |
| | 185,4 | 98,5 | ND | 44,91 | 2,53 |
| | 134,7 | 68,5 | 8 | 50,12 | 1,77 |
| | **194,2** | **94,8** | **4,2** | **48,16** | **2,45** |
| | **194,6** | **99,6** | **5,1** | **50,11** | **2,55** |
| Base saccharose pH non régulé en début de fermentation | 182,5 | 103,8 | 5 | 52,44 | 2,66 |

| | | | | | |
|---|---|---|---|---|---|
| c = consommé ; p = produit | | | | | |

Le rendement massique moyen basé sur les deux fermentations base mélasse de betterave est de 37,9 ± 0,2 %. Sa productivité est de 1,4 ± 0,05 g/L/h.

Les performances obtenues sur la base des trois meilleures fermentations base saccharose (en gras dans le tableau 1) sont les suivantes :
- Rendement massique : 48,2 ± 1,5 %
- Titre en Turanose : 99,3 ± 3,5 g/L (pour une base à 200 g/L de saccharose)
- Productivité : 2,55 ± 0,1 g/L/h

En 39 h de fermentation, tout le saccharose a été consommé. Il a été constaté lors des analyses que la souche I-4604 ne produit pas d'acide gluconique.

Il semblerait que les rendements massiques soient plus importants, lorsque que la souche I-4604 est cultivée dans des conditions où le pH n'est pas régulé au démarrage (le pH tombe à 5 après 14h de fermentation).

### 1.2- Fermentations réalisées avec la souche de référence ATCC 15928

| | **[Saccharose]_{c} *g.L⁻¹*** | **[Turanose]ₚ *g.L⁻¹*** | **[Isomaltulose]ₚ *g.L⁻¹*** | **Y_{Isom/Sac} %** | **Productivité *g.L⁻¹.h⁻¹*** |
|---|---|---|---|---|---|
| Base saccharose | 117,1 | 0 | 95 | 76,58 | 2,46 |
| | 209,8 | 0 | 138,7 | 63,46 | 3,59 |
| | 186,4 | 0 | 126,4 | 69,81 | 3,24 |

| | | | | | |
|---|---|---|---|---|---|
| c = consommé ; p = produit | | | | | |

Dans les mêmes conditions que la I-4604, cette souche ne produit pas du tout de turanose. En revanche, comme indiqué dans la littérature, elle produit de l'isomaltulose et un peu de tréhalulose (Kawaguti, et al, Food chemistry, 2010 120, no.3).

### 1.3- Fermentations réalisées avec les autres souches Serratia ficaria testées GRIMONT 4024 et DSM 4569

Elles ont montré une croissance similaire aux *S. plymuthica,* mais n'ont produit ni de turanose, ni d'isomaltulose.

### 2- Conclusions

Les souches ont été testées en triple dans les mêmes conditions de fermentation. Les résultats obtenus sont les suivants :

| | | | | |
|---|---|---|---|---|
| **Rendement Massique Turanose/Saccharose** | **Roquette I-4604** | **46,37 %** | **48,16 %** | **50,11 %** |
| | **ATCC15928** | **0%** | **0%** | **0%** |
| | **Grimont 4024** | **0%** | **0%** | **0%** |
| | **DSM 4569** | **0%** | **0%** | **0%** |

La souche I-4604 a été comparée à plusieurs souches de *Serratia,* toutes testées dans les mêmes conditions de production sur substrat saccharose. Seule la souche I-4604 produit du turanose.

### 3- Matériels et méthodes

### Mode opératoire

La préparation des échantillons comprenait trois étapes : une étape de revivification, une étape de préculture et une étape de subculture. La revivification comprenait trois repiquages successifs de la souche sur milieu gélosé. La préculture a été effectuée pendant 16 h à 160 rpm et 30°C dans le milieu de préculture. La subculture a été effectuée pendant 9 h à 160 rpm et 30°C dans le milieu de subculture.

Ensuite, une étape de fermentation a été effectuée dans des bioréacteurs DASGIP avec un volume de départ de 1500 mL. La fermentation a duré 39 h à 300 rpm, à 1 vvm (volume d'air par minute et par volume) (90 L/h) en maintenant le pH à 6 avec de la soude 5N.

### Composition des milieux

Milieu gélosé

| | |
|---|---|
| Saccharose: | 40 g/L |
| SOLULYS^{®} 048E: | 20 g/L |
| Peptone (Becton Dickinson) : | 10 g/L |
| Agar (Biokar Diagnostics) : | 20 g/L |
| Eau osmosée : | QSP 1000mL |

pH rectifié à 7 avec NaOH
Stérilisation 20min à 120°C
Milieu de préculture

| | |
|---|---|
| Saccharose: | 100 g/L |
| Stérilisation par filtration sur | 0.22µm |
| Extrait de levure: | 9,7 g/L |

pH rectifié à 7.0 (avec NaOH) / stérilisation 20 min à 120°C
1 goutte anti-mousse
Inoculum : 1 öse de 10 µl à partir du 3^{ème} repiquage
Milieu de subculture
Idem milieu de Préculture
Inoculum : 10 % de préculture (15 ml)
Milieu de production
1- Base de saccharose

| | |
|---|---|
| Saccharose | 200 g/L |
| Stérilisation par filtration sur | 0.22µm |
| Extrait de levure : 4,2 | g/L |
| pH rectifié à 7.0 (avec NaOH) / stérilisation 20 min à 120°C | 10 gouttes anti-mousse |

2- Base de mélasse de betterave (à 84,1 % DP2)

| | |
|---|---|
| Mélasse de betterave | 357 g |
| Stérilisation par filtration sur | 0.22µm |
| Extrait de levure: | 4,2 g/L |
| pH rectifié à 7.0 (avec NaOH) / stérilisation 20 min à 120°C | 10 gouttes anti-mousse |

Inoculum : 6 % subculture (90 ml)

### Exemple 2 - Optimisation des conditions de production du turanose base saccharose avec la souche I-4604

Plusieurs conditions d'agitation, d'aération et de pH ont été testées séparément lors de l'étape de production. Les milieux de préculture, subculture et production étaient les mêmes que dans l'exemple 1. Les optimisations ont été faites en Fermenteur de 2 L.

### Effet de l'agitation

L'agitation a été testée à 300, 500 et 700 rpm, les autres paramètres étant 30 °C, 1 vvm et un pH régulé à 6. Les résultats ont été les suivants.

**300 rpm**

| | | | | |
|---|---|---|---|---|
| Temps (h) | 0 | 15 | 22,5 | 39,5 |
| Saccharose (g/l) | 198,1 | 74,2 | 33,9 | 2,3 |
| Turanose (g/l) | 3 | 76 | 98,3 | 113,8 |
| Conso. saccharose (g/l/h) | - | 8,26 | 5,37 | 1,86 |
| Prod. turanose (g/l/h) | - | 4,87 | 2,97 | 0,91 |
| Rendement turanose (%) | - | - | **58,04** | - |

**500 rpm**

| | | | | |
|---|---|---|---|---|
| Temps (h) | 0 | 15 | 22,5 | 39,5 |
| Saccharose (g/l) | 193,2 | 68,5 | 32,1 | 4,7 |
| Turanose (g/l) | 2,8 | 70,8 | 90,7 | 103,1 |
| Conso. saccharose (g/l/h) | - | 8,31 | 4,85 | 1,61 |
| Prod. turanose (g/l/h) | - | 4,53 | 2,65 | 0,73 |
| Rendement turanose (%) | - | - | **54,56** | - |

**700 rpm**

| | | | | |
|---|---|---|---|---|
| Temps (h) | 0 | 15 | 22,5 | 39,5 |
| Saccharose (g/l) | 202,2 | 67,3 | 31,9 | 4,6 |
| Turanose (g/l) | 2,8 | 72,8 | 93,1 | 107,7 |
| Conso. saccharose (g/l/h) | - | 8,99 | 4,72 | 1,61 |
| Prod. turanose (g/l/h) | - | 4,67 | 2,71 | 0,86 |
| Rendement turanose (%) | - | - | **53,02** | - |

### Effet de l'aération

L'aération a été testée à 0,5, 1 et 1,5 vvm, les autres paramètres étant 30 °C, 300 rpm et un pH régulé à 6. Les résultats ont été les suivants.

**0,5 vvm**

| | | | |
|---|---|---|---|
| Temps (h) | 0 | 14,25 | 22,75 |
| Saccharose (g/l) | 196,2 | 64,1 | 19,7 |
| Turanose (g/l) | 3,3 | 74,4 | 100 |
| Conso. saccharose (g/l/h) | - | 9,27 | 5,22 |
| Prod. turanose (g/l/h) | - | 4,99 | 3,01 |
| Rendement turanose (%) | - | - | **54,79** |

**1 vvm**

| | | | |
|---|---|---|---|
| Temps (h) | 0 | 14,25 | 22,75 |
| Saccharose (g/l) | 189,6 | 74,3 | 26,8 |
| Turanose (g/l) | 2,9 | 71,3 | 98,9 |
| Conso. saccharose (g/l/h) | - | 8,09 | 5,59 |
| Prod. turanose (g/l/h) | - | 4,80 | 3,25 |
| Rendement turanose (%) | - | - | **58,97** |

**1,5 vvm**

| | | | |
|---|---|---|---|
| Temps (h) | 0 | 14,25 | 22,75 |
| Saccharose (g/l) | 201,6 | 74,7 | 26,3 |
| Turanose (g/l) | 3,5 | 76,3 | 104,4 |
| Conso. saccharose (g/l/h) | - | 8,91 | 5,69 |
| Prod. turanose (g/l/h) | - | 5,11 | 3,31 |
| Rendement turanose (%) | - | - | **57,56** |

### Effet du pH

Le pH a été testé à 5,5, 6 et 6,5, les autres paramètres étant 30 °C, 300 rpm et 1 vvm. Les résultats ont été les suivants.

**pH régulé à 5,5**

| | | | |
|---|---|---|---|
| Temps (h) | 0 | 15 | 23 |
| Saccharose (g/l) | 194,3 | 74,7 | 32,8 |
| Turanose (g/l) | 3,7 | 69,4 | 89,9 |
| Conso. saccharose (g/l/h) | - | 7,97 | 5,24 |
| Prod. turanose (g/l/h) | - | 4,38 | 2,56 |
| Rendement turanose (%) | - | - | **53,37** |

**pH régulé à 6**

| | | | |
|---|---|---|---|
| Temps (h) | 0 | 15 | 23 |
| Saccharose (g/l) | 198,1 | 62,2 | 24,3 |
| Turanose (g/l) | 3,4 | 82,5 | 105 |
| Conso. saccharose (g/l/h) | - | 9,06 | 4,74 |
| Prod. turanose (g/l/h) | - | 5,27 | 2,81 |
| Rendement turanose (%) | - | - | **58,46** |

**pH régulé à 6,5**

| | | | |
|---|---|---|---|
| Temps (h) | 0 | 15 | 23 |
| Saccharose (g/l) | 197,9 | 64,2 | 27,2 |
| Turanose (g/l) | 3,5 | 77,1 | 99,7 |
| Conso. saccharose (g/l/h) | - | 8,91 | 4,63 |
| Prod. turanose (g/l/h) | - | 4,91 | 2,83 |
| Rendement turanose (%) | - | - | **56,36** |

Les conditions les plus adéquates ont été définies comme une agitation de 300 rpm, une aération de 1 vvm et un pH régulé à 6,0.

En outre, la quantité de saccharose mise en oeuvre lors de l'étape de fermentation ainsi que le milieu de culture utilisé ont fait l'objet d'optimisation.

### Effet de la concentration en saccharose de départ

Deux concentrations de départ ont été testées, à savoir 100 et 200 g/L de saccharose.

Une préculture a été réalisée à 30°C, 160 rpm, pendant 23 h 30 dans un milieu à un pH 7 comprenant 30 g/L de saccharose et 30 g/L de SOLULYS^{®} 048E stérilisé séparément 20 min à 120°C et 1 goutte antimousse. La production a été réalisée à 30°C, 300 rpm, 1 vvm pendant 25 h dans un milieu à un pH 7 comprenant 100 ou 200 g/L de saccharose et 30 g/L de SOLULYS^{©} 048E. stérilisé séparément 20 min à 120°C et 10 gouttes antimousse. Le pH est relevé à 5,8 à 6 h de fermentation et régulé à 5,5 à partir de 9 h.

**100 g/L de saccharose de départ**

| | | | | |
|---|---|---|---|---|
| Temps (h) | 0 | 6 | 9 | 25 |
| Saccharose (g/l) | 101,6 | 45,3 | 16,9 | <0,5 |
| Turanose (g/l) | 2,1 | 28,1 | 40,7 | 35,1 |

**200 g/L de saccharose de départ**

| | | | | |
|---|---|---|---|---|
| Temps (h) | 0 | 6 | 9 | 25 |
| Saccharose (g/l) | 190,3 | 150,6 | 117,7 | 11 |
| Turanose (g/l) | 1,9 | 26,2 | 44,8 | 92,9 |

La concentration de saccharose de départ de 200 g/L donne de meilleurs résultats.

### Etude du milieu

Trois groupes de milieux de culture ont été testés.

| | Préculture | Subculture | Production |
|---|---|---|---|
| Milieu 1 | Saccharose 100 g/L | Saccharose 100 g/L | Saccharose 200 g/L |
| | Extrait de levure 14 g/L | Extrait de levure 10 g/L | NH₄H₂PO₄ 1,3 g/L |
| | SOLULYS^{®} 048E. 5 g/L | SOLULYS^{®} 048E. 5 g/L | (NH₄)₂HPO₄ 1,3 g/L |
| | | | MgSO₄, 7H₂O 0,4 g/L |
| | | | Fe(NH4)2(SO4)2 0, g/L |
| Milieu 2 | Saccharose 100 g/L | Saccharose 100 g/L | Saccharose 200 g/L |
| | Extrait de levure 9,7 g/L | Extrait de levure 9,7 g/L | Extrait de levure 4,2 g/L |
| Milieu 3 | Saccharose 100 g/L | Saccharose 100 g/L | Saccharose 200 g/L |
| | SOLULYS^{®} 048E 30 g/L | SOLULYS^{®} 048E 30 g/L | SOLULYS^{©} 048E 13 g/L |
| Conditions | 30°C, 165 rpm, 18 h | 30°C, 165 rpm, 9 h | 30°C, 300rpm, pH 6 |

Les résultats obtenus sont les suivants.

| | Temps (h) | 0 | 15 | 20,5 | 24 | 39,5 |
|---|---|---|---|---|---|---|
| Milieu 1 (g/L) | saccharose | 204 | 75,8 | 45,3 | 28,8 | 1,1 |
| | turanose | - | 70,6 | 88,5 | 102,6 | 111,8 |
| Milieu 2 (g/L) | saccharose | 204,8 | 66,8 | 37,7 | 22,7 | 1 |
| | turanose | - | 78,8 | 96,9 | 104,8 | 114,5 |
| Milieu 3 (g/L) | saccharose | 193,8 | 94,2 | 58,9 | 44,7 | 7,8 |
| | turanose | - | 65,8 | 79 | 90,9 | 113,2 |

Le milieu 2 semble le plus approprié car il permet un gain de temps.

### Exemple 3 - Production de turanose à l'aide de la souche I-4604

La production de turanose à l'aide de la souche I-4604 dans des fermenteurs de 20 L a été réalisée.

La fermentation a été effectuée à partir de saccharose et de mélasse. Le protocole était le suivant.

**Préculture**

| | |
|---|---|
| Saccharose | 100 g/L |
| SOLULYS^{®} 048E | 15 g/L |
| Erol 18 | 1 goutte |

Le tout, stérilisé à 120°C pendant 20 min et ajusté à pH 7.

La préculture est réalisée dans 3 erlens de 500 mL pendant 24 h à 30°C et 120 rpm.

**Subculture**

| | |
|---|---|
| Saccharose | 100 g/L |
| SOLULYS^{®} 048E | 30 g/L |
| Erol 18 | 0,5 ml/L |

Le tout, stérilisé à 120°C pendant 20 min et ajusté à pH 7.

La subculture est réalisée en fermenteur dans un volume de 15 L pendant 19 h à 30°C 300 rpm et 1 vvm.

**Production**

| | |
|---|---|
| Saccharose | 100 g/L ) stérilisé à 120°C pendant 10 min |
| Erol 18 | 0,5 ml/L ) idem |
| SOLULYS^{©} 048E | 30 g/L stérilisé à 120°C pendant 20 min |

Le pH est ajusté à pH 6. La culture est réalisée en fermenteur dans un volume de 15 L à 30°C 300 rpm et 1 vvm.

**Résultats**

| Temps (h) (g/L) | 14 | 22 | 30 | 36 | 47 |
|---|---|---|---|---|---|
| Saccharose | 45,9 | 11,7 | 1,3 | 0 | 0 |
| Glucose | 0 | 0 | 0 | 0 | 0 |
| Fructose | 17,1 | 18,3 | 14,5 | 7,3 | 0 |
| Isomaltulose | 4,1 | 4,5 | 4,8 | 4,9 | 4,8 |
| Tréhalulose | 9,5 | 10,7 | 11,1 | 11,8 | 11,1 |
| **Turanose** | **93,9** | **97,3** | **97,7** | **102,1** | **102,8** |
| Richesse | 75,3 | 74,4 | 76,3 | 80,9 | 86,6 |
| Rendement (%) | 60,9 | 51,7 | 49,1 | 51,05 | 51,4 |

Les résultats moyens sur 4 essais sont les suivants :
- Rendement (turanose/saccharose) : 53 %
- Durée : 45 à 50 h
- Composition finale en sucres :
   ▪ Turanose : 106 g/L (soit 86 % des sucres)
   ▪ Tréhalulose : 12 g/L
   ▪ Isomaltulose : 5 g/L

Le milieu de culture proposé initialement étant simple et peu onéreux, les effets de la température, du pH, du niveau d'oxygénation et du mode d'apport du saccharose ont ensuite été étudiés.

Le tableau ci-dessous récapitule les résultats de l'ensemble des essais effectués :

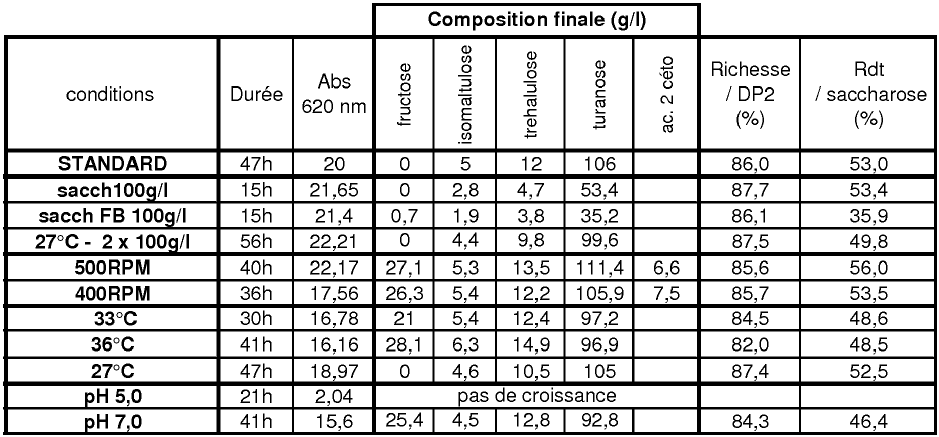

Aucune modification n'a eu d'effet significativement positif mais on peut cependant tirer les conclusions suivantes :
- le pH doit être maintenu à 6,0 ;
- la température doit être maintenue en dessous de 30°C. L'optimum semble même proche de 27 °C.
- un niveau d'oxygénation trop important (agitation de 400 ou 500 rpm) ralentit la consommation du saccharose et entraîne une forte production d'acide 2-cétogluconique.
- l'ajout d'une deuxième étape de préculture n'apporte pas d'avantage.
- il n'est pas intéressant d'apporter le saccharose progressivement (FB = fed-batch).

### Exemple 4 - Purification du turanose à partir du mout de fermentation de la souche I-4604

### 1- Les étapes de purification du mout de fermentation

La séparation de la biomasse a été effectuée par centrifugation à 12 000 g. Le surnageant était encore trouble. Pour enlever ce trouble, un traitement au noir en poudre (1 % de SX+) et une filtration sur plaque Cofram EKS ont été effectués. Le filtrat obtenu était plus limpide, mais non décoloré.

La déminéralisation a été réalisée sur cation fort (C150) et anion faible (4228). La charge du produit avant déminéralisation était de 170 meq/l. La résistivité du produit purifié était inférieure à 5 kOhm car il contenait plusieurs acides organiques. La composition en acides organiques est indiquée dans les tableaux ci-dessous.

Pour améliorer la richesse avant cristallisation, une ultrafiltration a été réalisée sur 1 kD. Cette étape a permis de retenir la coloration et les produits de poids moléculaires supérieurs à 1 kD.

### 2- Cristallisation

La cristallisation a été réalisée à température ambiante, après avoir porté à ébullition un mélange de 100 g de produit à 70 bx et 200 mL d'éthanol 96 %. La séparation des cristaux et des eaux-mères a été effectuée sur Büchner étant donnée la faible viscosité du mélange. Dans ces conditions, pour 2 litres de fermenteur mis en oeuvre, 70 g de cristaux (richesse en turanose 98-99 %) ont été récupérés, soit un rendement massique de 30 %.

Les cristaux présentaient une bonne richesse, comme l'illustre les tableaux ci-dessous.

| **Résultats en g/L** | |
|---|---|
| Turanose | 115,6 |
| Tréhalulose | 14 |
| Isomaltulose | 6,3 |
| Ac. glycérique glucosylé | ∼5 |
| Fructose | 2,8 |
| Ac 2-cétoglutarique | ∼2 |
| Ac citrique | ∼2 |
| Ac succinique | ∼1 |
| Ac lactique glucosylé | ∼3 |

| **Résultats en %/brut** | |
|---|---|
| Matière sèche | 99,8 |
| Fructose | <0,05 |
| Glucose | <0,05 |
| Ac lactique glucosylé | <0,05 |
| Palatinose | 1,1 |
| tréhalulose | 0,2 |
| Autres DP2 | 0,3 |

Ces différentes étapes de purification ont permis d'obtenir du turanose de bonne richesse.

## Revendications

1. Souche de *Serratia plymuthica* déposée le 7 mars 2012 auprès de la CNCM sous le numéro I-4604.

2. Souche de *Serratia plymuthica* **caractérisée en ce qu'**elle est capable de produire du turanose, et qu'elle est obtenue à partir de la souche selon la revendication 1 par culture, mutagenèse ou modification génétique de celle-ci.

3. Souche selon l'une des revendications 1 ou 2, **caractérisée en ce qu'**elle est capable de produire du turanose avec un rendement massique turanose/saccharose d'au moins 20 %, 30 %, 40 % ou 50 %.

4. Utilisation de la souche selon l'une des revendications 1 à 3 pour produire du turanose.

5. Méthode de production de turanose comprenant une culture de la souche selon l'une quelconque des revendications 1 à 3 et la récupération du turanose, et facultativement la purification du turanose.

6. Méthode selon la revendication 5, **caractérisée en ce que** la souche est cultivée à pH compris entre 5,5 et 7, une aération de 0,5 à 1,5 vvm, dans des conditions d'agitation comprise entre 250 et 700 rpm, et à une température de 25 à 38 °C.

7. Méthode selon la revendication 5 ou 6, **caractérisée en ce que** la souche est cultivée à un pH maintenu à 6, une température de 27 à 30°C, des conditions d'agitation comprises entre 250 et 350 rpm, et une aération de 1 vvm.

8. Méthode selon l'une quelconque des revendications 5 à 7, dans laquelle la concentration de saccharose de départ dans le milieu de production est comprise entre 100 et 300 g/L, de préférence de 200 g/L.

9. Méthode selon l'une quelconque des revendications 5 à 8, dans laquelle le milieu de production comprend 2 à 6 g/L d'extrait de levure ou 10 à 30 g/L de SOLULYS^{®} 048E, de préférence environ 4 g/L d'extrait de levure.

10. Méthode selon l'une quelconque des revendications 5 à 9, dans laquelle la purification du turanose comprend une étape de centrifugation du mout de fermentation, des étapes de traitement au noir en poudre et de filtration du surnageant, une étape de déminéralisation, une étape d'ultrafiltration avec un seuil de coupure à 1 kD, puis une étape de cristallisation du filtrat.

## Patentansprüche

1. *Serratia plymuthica* Stamm, hinterlegt am 7. März 2012 bei der CNCM unter der Hinterlegungsnummer I-4604.

2. *Serratia plymuthica* Stamm, **dadurch gekennzeichnet, dass** er in der Lage ist, Turanose zu produzieren, und dass er ausgehend von dem Stamm gemäß Anspruch 1 durch Kultivierung, Mutagenese oder genetische Modifikation besagten Stammes erhalten wird.

3. Stamm gemäß einem der Ansprüche 1 oder 2, **dadurch gekennzeichnet, dass** er in der Lage ist, Turanose mit einer Turanose/Saccharose-Massenausbeute von wenigstens 20%, 30%, 40% oder 50% zu produzieren.

4. Verwendung des Stammes gemäß einem der Ansprüche 1 bis 3 zur Produktion von Turanose.

5. Verfahren zur Produktion von Turanose, umfassend Kultivierung des Stammes gemäß einem der Ansprüche 1 bis 3 und Wiedergewinnung der Turanose und gegebenenfalls Reinigung der Turanose.

6. Verfahren gemäß Anspruch 5, **dadurch gekennzeichnet, dass** der Stamm bei einem pH zwischen 5,5 und 7, einer Belüftung von 0,5 bis 1,5 vvm, unter Rührbedingungen zwischen 250 und 700 rpm und bei einer Temperatur von 25 bis 38°C kultiviert wird.

7. Verfahren gemäß Anspruch 5 oder 6, **dadurch gekennzeichnet, dass** der Stamm bei einem pH, der auf einen Wert von 6 gehalten wird, einer Temperatur von 27 bis 30°C, Rührbedingungen zwischen 250 und 350 rpm und einer Belüftung von 1 vvm kultiviert wird.

8. Verfahren gemäß einem der Ansprüche 5 bis 7, wobei die anfängliche Saccharose-Konzentration im Produktionsmedium zwischen 100 und 300 g/l, vorzugsweise 200 g/l, beträgt.

9. Verfahren gemäß einem der Ansprüche 5 bis 8, wobei das Produktionsmedium 2 bis 6 g/l Hefeextrakt oder 10 bis 30 g/l SOLULYS^{©} 048E, vorzugsweise ungefähr 4 g/l Hefeextrakt, umfasst.

10. Verfahren gemäß einem der Ansprüche 5 bis 9, wobei die Reinigung der Turanose einen Schritt der Zentrifugation der Fermentationsbrühe, Schritte der Behandlung mit Kohlepulver und der Filtration des Überstands, einen Schritt der Demineralisation, einen Schritt der Ultrazentrifugation mit einer Ausschlussgrenze von 1 kD, anschließend einen Schritt der Kristallisation des Filtrats umfasst.

## Claims

1. A *Serratia plymuthica* strain deposited on March 7, 2012, with the CNCM under number I-4604.

2. A *Serratia plymuthica* strain **characterized in that** it is capable of producing turanose, and **in that** it is obtained from the strain as claimed in claim 1 by culturing, mutagenesis or genetic modification of said strain.

3. The strain as claimed in either of claims 1 and 2, **characterized in that** it is capable of producing turanose with a turanose/sucrose weight yield of at least 20%, 30%, 40% or 50%.

4. The use of the strain as claimed in one of claims 1 to 3, for producing turanose.

5. A method for producing turanose, comprising culturing the strain as claimed in any one of claims 1 to 3 and recovering the turanose, and optionally purifying the turanose.

6. The method as claimed in claim 5, **characterized in that** the strain is cultured at a pH of between 5.5 and 7, an aeration of from 0.5 to 1.5 vvm, under agitation conditions of between 250 and 700 rpm, and at a temperature of from 25 to 38°C.

7. The method as claimed in claim 5 or 6, **characterized in that** the strain is cultured at a pH maintained at 6, a temperature of from 27 to 30°C, under agitation conditions of between 250 and 350 rpm, and an aeration of 1 vvm.

8. The method as claimed in any one of claims 5 to 7, in which the starting sucrose concentration in the production medium is between 100 and 300 g/l, preferably 200 g/l.

9. The method as claimed in any one of claims 5 to 8, in which the production medium comprises 2 to 6 g/l of yeast extract or 10 to 30 g/l of Solulys® 048E, preferably approximately 4 g/l of yeast extract.

10. The method as claimed in any one of claims 5 to 9, in which the purification of the turanose comprises a fermentation must centrifugation step, steps of treatment with powdered black carbon and of filtration of the supernatant, a demineralization step, a step of ultrafiltration with a cut-off threshold of 1 kD, and then a filtrate crystallization step.
